(19) Europäisches Patentamt
European Patent Office
Office européen des brevets

(11) Veröffentlichungsnummer: **0 384 311 A1**

(12) **EUROPÄISCHE PATENTANMELDUNG**

(21) Anmeldenummer: **90103017.1**

(51) Int. Cl.5: **C07D 213/84, A01N 43/42**

(22) Anmeldetag: **16.02.90**

(30) Priorität: **21.02.89 DE 3905238**

(43) Veröffentlichungstag der Anmeldung:
**29.08.90 Patentblatt 90/35**

(84) Benannte Vertragsstaaten:
**AT BE CH DE DK ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Schubert, Juergen, Dr.**
**Am Oberen Luisenpark 2**
**D-6800 Mannheim 1(DE)**
Erfinder: **Wild, Jochen, Dr.**
**An der Marlach 7**
**D-6705 Deidesheim(DE)**
Erfinder: **Harreus, Albrecht, Dr.**
**Teichgasse 13**
**D-6700 Ludwigshafen(DE)**
Erfinder: **Kuekenhoehner, Thomas, Dr.**
**Seidelstrasse 2**
**D-6710 Frankenthal(DE)**
Erfinder: **Sauter, Hubert, Dr.**
**Neckarpromenade 20**
**D-6800 Mannnheim 1(DE)**
Erfinder: **Ammermannn, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

(54) **2-Anilino-cyanopyridine und diese enthaltende Fungizide.**

(57) 2-Anilino-cyanopyridine der Formel,

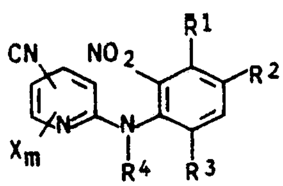

in welcher
X
$NO_2$, CN, Halogen, $SO_2$-Alkyl, Alkyl, Cycloalkyl, Haloalkyl, Alkoxy, gegebenenfalls im Phenylrest substituiertes Phenoxy oder Phenylthio steht,
m
eine ganze Zahl von 1-3 ist,
$R^1$
Wasserstoff, Halogen, Alkoxy, Halogenalkyl, Halogenalkoxy, gegebenenfalls im Phenylrest substituiertes Phenoxy oder Phenylthio ist,
$R^2$, $R^3$
Wasserstoff, $NO_2$, Halogen, CN, Alkyl, $SO_2$-Alkyl, Halogenalkyl, $SO_2NR^5R^6$, Halogenalkoxy, $COOR^5$, $CONR^5R^6$ ist,
$R^4$
Wasserstoff, $COOR^7$, $CONR^5R^6$, CHO, $COR^7$, $SO_2R^7$, $C_3$-$C_6$-Alkenyl, $C_3$-$C_6$-Alkinyl ist,
und Salze der 2-Anilino-cyanopyridine, in denen $R^4$ Wasserstoff bedeutet, mit pflanzenverträglichen

EP 0 384 311 A1

Kationen und diese Verbindungen enthaltende Fungizide.

## 2-Anilino-cyanopyridine und diese enthaltende Fungizide

Die vorliegende Erfindung betrifft neue, wertvolle 2-Anilino-cyanopyridine, Verfahren zu ihrer Herstellung, diese Verbindungen enthaltende fungizide Mittel und ihre Verwendung als Fungizide.

Es ist bekannt, daß 2-Anilino-pyridine, zum Beispiel das 2-(2,4-Dinitro-6-trifluormethylanilino)-5-trifluormethylpyridin (EP A-31 257) eine gute fungizide Wirkung zeigen. Die Wirkung ist jedoch bei niedrigen Aufwandmengen und Anwendungskonzentrationen nicht immer befriedigend.

Überraschend wurde nun gefunden, daß 2-Anilino-cyanopyridine der Formel I

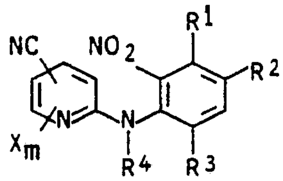

I

in welcher

X

$NO_2$, CN, Halogen, $SO_2$-$C_1$-$C_4$-Alkyl, $C_1$-$C_4$-Alkyl, $C_3$-$C_6$-Cycloalkyl, $C_1$-$C_4$-Halogenalkyl, $C_1$-$C_4$-Alkoxy, gegebenenfalls im Phenylrest substituiertes Phenoxy oder Phenylthio steht,

m

eine ganze Zahl von 1-3 ist, wobei die einzelnen Reste gleich oder verschieden sind, wenn m größer als 1 ist,

$R^1$

Wasserstoff, Halogen, $C_1$-$C_4$-Alkoxy, $C_1$-$C_4$-Halogenalkyl, $C_1$-$C_4$-Halogenalkoxy, gegebenenfalls im Phenylrest substituiertes Phenoxy oder Phenylthio ist,

$R^2$, $R^3$

unabhängig voneinander Wasserstoff, $NO_2$, Halogen, CN, $C_1$-$C_6$-Alkyl, $SO_2$-$C_1$-$C_4$-Alkyl, $C_1$-$C_6$-Halogenalkyl, $SO_2NR^5R^6$, $C_1$-$C_4$-Halogenalkoxy, $COOR^5$, $CONR^5R^6$ ist,

$R^4$

Wasserstoff, $COOR^7$, $CONR^5R^6$, CHO, $COR^7$, $SO_2R^7$, $C_3$-$C_6$-Alkenyl, $C_3$-$C_6$-Alkinyl ist,

$R^5$, $R^6$

unabhängig voneinander Wasserstoff, $C_1$-$C_4$-Alkyl ist,

$R^7$

$C_1$-$C_4$-Alkyl, $C_1$-$C_4$-Halogenalkyl, Benzyl, gegebenenfalls substituiertes Aryl ist,

und Salze der 2-Anilino-cyanopyridine der Formel I, in der $R^4$ Wasserstoff bedeutet, mit pflanzenverträglichen Kationen eine sehr gute fungizide Wirkung bei ausgezeichneter Verträglichkeit bei Pflanzen haben.

Unter dem Begriff Alkyl selbst oder als Bestandteil eines anderen Substituenten, wie Halogenalkyl, Alkoxy, Halogenalkoxy sind je nach Zahl der angegebenen Kohlenstoffatome die folgenden geradkettigen oder verzweigten Gruppen zu verstehen: Methyl, Ethyl, n-Propyl, iso-Propyl, n-Butyl, sec.-Butyl, iso-Butyl, tert.-Butyl, n-Pentyl, n-Hexyl. Die Vorsilbe Halogen in der Bezeichnung eines Substituenten bedeutet hier und im folgenden, daß dieser Substituent einfach bis perhalogeniert auftreten kann. Halogen steht stellvertretend für F, Cl, Br oder J. Halogenalkyl steht somit für einen einfach bis perhalogenierten Alkylrest, wie $CH_2Cl$, $CHCl_2$, $CCl_3$, $CH_2F$, $CHF_2$, $CF_3$, $CH_2Br$, CHBrCl, $CF_2Cl$, $C_2Cl_5$, $C_2F_5$, $CF_2$-$CHF_2$, $CH_2CH_2Cl$, $CH_2CH_2Br$, $C_3F_7$, $C_4F_9$.

Cycloalkyl steht für Cyclopropyl, Cyclobutyl, Cyclopentyl und Cyclohexyl, wobei die Ringe ein- bis zweifach methylsubstituiert sein können, wie z. B. 1-Methylcylopropyl, 2-Methylcyclopropyl, 2,2-Dimethylcyclopropyl, 1-Methylcyclopentyl, 1-Methylcyclohexyl.

Alkenyl steht z. B. für Allyl, 3-Butenyl, 4-Butenyl, trans-2-Butenyl, cis-2-Butenyl.

Alkinyl steht z. B. für Propargyl, 3-Butinyl, 4-Butinyl, 5-Pentinyl.

Gegebenenfalls substituiertes Aryl steht z. B. für Phenyl, 2-, 3-, 4-Tolyl, Halogenphenyl, 4-Brom-phenyl, 4-Chlor-phenyl, 4-Fluor-phenyl, Nitrophenyl, 2-Nitrophenyl, 4-Nitrophenyl, 1- und 2-Naphthyl.

Gegebenenfalls substituiertes Phenoxy oder Phenylthio bedeutet, daß der aromatische Ring unsubstituiert ist oder ein- bis dreifach halogeniert sein kann, wie 2-Cl, 2-F, 3-Cl, 4-Cl, 4-Br, 2,3-$Cl_2$, 2,4-$Cl_2$, 2,5-$Cl_2$, 2,6-$Cl_2$, 3,5-$Cl_2$, 2,4,6-$Cl_3$, 3,4,5-$Cl_3$ oder ein bis zwei Substituenten wie Nitro oder Methyl, 2-$NO_2$, 4-$NO_2$, 2-$CH_3$, 4-$CH_3$, 2,6-$(CH_3)_2$, 3,5-$(CH_3)_2$ tragen kann.

Für die Salze kommen beispielsweise folgende Kationen in Betracht: $Na^+$, $K^+$, $Mg^{2+}$, $Fe^{3+}$, $NH_4^+$,

einfach oder mehrfach alkylierte, hydroxyalkylierte und/oder arylierte Ammoniumkationen, wie Diisopropylammonium, Tetramethylammonium, Tetrabutylammonium, Trimethylbenzylammonium, N,N-Dimethylanilinium, Trimethyl-(2-hydroxyethyl)ammonium, wobei das Alkyl 1 bis 4 C-Atome hat und der Arylrest ein Phenylrest oder ein Benzylrest ist.

Die Verbindungen der Formel I gemäß Anspruch 1, in der $R^4$ Wasserstoff ist, werden z. B. dadurch hergestellt, daß man ein Cyanopyridin der Formel II,

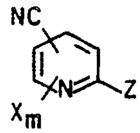

II,

in der X und m die in Anspruch 1 angegebene Bedeutung haben, mit Verbindungen der Formel III

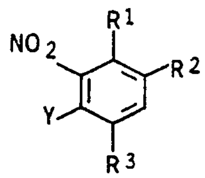

III,

in der $R^1$, $R^2$, $R^3$ die in Anspruch 1 angegebene Bedeutung haben, in Substanz oder in einem Lösungs- oder Verdünnungsmittel, gegebenenfalls in Anwesenheit einer organischen oder anorganischen Base bei Temperaturen zwischen -20 und 200°C umsetzt, wobei Y und Z für $NH_2$ oder Halogen stehen und Y und Z voneinander verschieden sind.

In beiden Fällen erfolgt die Umsetzung unter Abspaltung von Halogenwasserstoff. In beiden Fällen ist deshalb die Verwendung von säurebindenden Mitteln bzw. Basen von Vorteil.

Als Basen bzw. säurebindende Mittel können organische oder anorganische Verbindungen verwendet werden, wie z. B. Alkali- und Erdalkalihydroxide (LiOH, NaOH, KOH, $Ca(OH)_2$), -oxide ($Na_2O$ $Li_2O$, CaO, MgO), -hydride (LiH, NaH, KH, $CaH_2$), -amide ($LiNH_2$, $NaNH_2$, $KNH_2$), -carbonate ($Li_2CO_3$, $Na_2CO_3$, $CaCO_3$), -hydrogencarbonate ($NaHCO_3$), -alkyle (ButylLi, $CH_3Li$, $CH_3MgCl$), -$C_1$-$C_5$-alkoholate ($NaOCH_3$, $NaOC_2H_5$, $KOC_2H_5$, KO-tert.-Butyl, $Mg(OCH_3)_2$), Amine, insbes. tertiäre Amine (z. B. Trimethylamin, Triethylamin, Diisopropylethylamin, N-Methylpiperidin), Pyridin, substituierte Pyridine (Collidin, Lutidine, 4-Dimethylaminopyridin), bicyclische Amine.

Die Reaktionen können in Anwesenheit von reaktionsinerten Lösungs-oder Verdünnungsmitteln durchgeführt werden.

In Frage kommen beispielsweise aliphatische und aromatische Kohlenwasserstoffe wie Toluol, Xylole, Cyclohexan, Petrolether; halogenierte Kohlenwasserstoffe wie Chlorbenzol, Methylenchlorid, Chloroform; Ether und etherartige Verbindungen wie Dialkylether (Diethylether, Diisopropylether, tert.-Butylmethylether, usw.), Anisol, Dioxan, Tetrahydrofuran; Nitrile wie Acetonitril, Propionitril, N,N-dialkylierte Amide wie Dimethylformamid; Dimethylsulfoxid; Ketone wie Aceton, Diethylketon, Methylethylketon, Alkohole wie Methanol, Ethanol, n- und iso-Propanol, Butanole, insbesondere tert.-Butanol und Gemische solcher Lösungsmittel untereinander.

Die Reaktionstemperatur läßt sich in weiten Grenzen ändern. Je nach Art der Substituenten X, $R^1$, $R^2$, $R^3$, $R^5$, $R^6$, $R^7$ und ihrer Zahl m arbeitet man vorteilhaft bei Temperaturen zwischen -20°C und 200°C bzw. am Siedepunkt des Lösungsmittels bzw. Lösungsmittelgemisches.

In manchen Fällen kann es auch von Vorteil sein, die Reaktion unter Schutzgasatmosphäre und/oder wasserfreien Lösungsmitteln durchzuführen. Als Schutzgas eignen sich inerte Gase wie Helium, Argon usw., bevorzugt Stickstoff.

Die Ausgangsverbindungen sind im Handel erhältlich, bekannt oder nach bekannten Methoden einfach herstellbar. Die eingesetzten Halogenaromaten (bzw. einfach in diese umwandelbare Phenole) und Aniline sind im Handel erhältlich.

Für 2-Amino- bzw. 2-Halogen-cyanopyridine seien folgende Literaturstellen genannt: DE-A 31 03 065 und DE-A 35 28 459.

Noch nicht beschriebende 2-Amino- bzw. 2-Halogen-cyanopyridine können mit bekannten chemischen Synthesemethoden aus substituierten 2-Hydroxypyridinen bzw. substituierten Pyridincarbonsäuren hergestellt werden.

z.B. 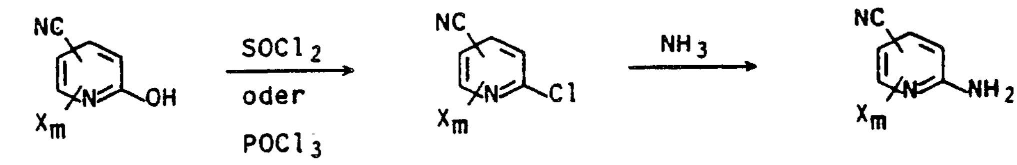


oder 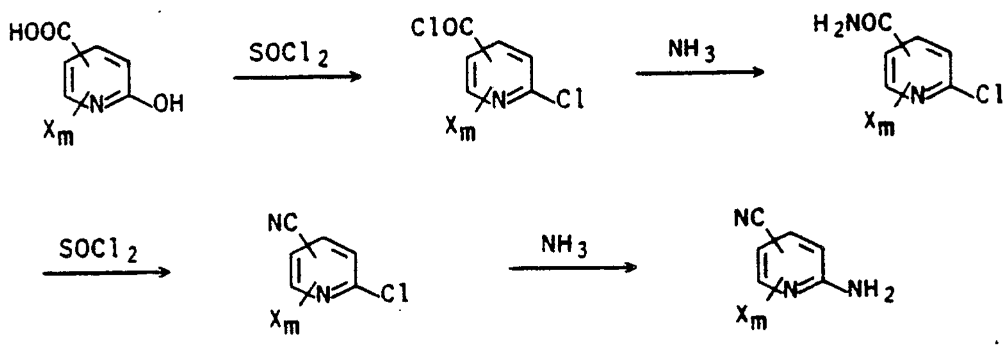


Die Verbindungen der Formel I, gemäß Anspruch 1 mit der Maßgabe, daß $R^4$ verschieden von Wasserstoff ist, werden z. B. dadurch hergestellt, daß man ein Cyanopyridin der Formel I, in der $R^4$ Wasserstoff ist und X, m, $R^1$, $R^2$, $R^3$, $R^5$, $R^6$, $R^7$ die in Anspruch 1 angegebene Bedeutung haben, mit einem Alkylierungs-, Acylierungs- oder Sulfonylierungsmittel der Formel IV,

$LR^4$ IV

in der

R4 die in Anspruch 1 angegebene Bedeutung außer Wasserstoff hat und L für eine nucleophil verdrängbare Abgangsgruppe steht, in Substanz oder in Gegenwart eines Lösungs- oder Verdünnungsmittels und gegebenenfalls in Gegenwart einer organischen oder anorganischen Base bei Temperaturen zwischen -20°C und 150°C umsetzt.

Als nucleophil verdrängbare Gruppen eignen sich bevorzugt Halogenatome wie F, Cl, Br, J, Sulfonate (Methanolsulfonat, Benzolsulfonat u. a.), Alkoholate (Ethylat, Benzylat u. a.) und Carboxylate (Acetat u. a.).

Als Basen können die gleichen Verbindungen wie bei der Herstellung von Verbindungen der Formel I ($R^4$ = H) aus Verbindungen der Formeln II und III verwendet werden.

Die Reaktionstemperaturen liegen i. a. zwischen -20°C und 150°C bzw. am Siedepunkt des Lösungsmittels oder Lösungsmittelgemisches.

Bei der Reaktion können ein oder mehrere reaktionsinerte Lösungs-oder Verdünnungsmittel anwesend sein. In Frage kommen beispielsweise aliphatische und aromatische Kohlenwasserstoffe wie Toluol, Xylole, Cyclohexan, Petrolether; halogenierte Kohlenwasserstoffe wie Chlorbenzol, Methylenchlorid, Ethylenchlorid, Chloroform, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Zur Isolierung der neuen Verbindungen wird nach üblichen Methoden vorgegangen. Die anfallenden Produkte können durch Umkristallisieren, Extrahieren oder Chromatographieren gereinigt werden. Folgende Beispiele sollen die Herstellung illustrieren:

Beispiel 1

Zu einer Suspension von 57,5 g (0,25 Mol) 2-Amino-3,5-dicyano-6-ethoxy-4-isopropyl-pyridin und 83,8 g (0,275 Mol) 3-Chlor-2,6-dinitro-4-trifluormethyl-chlorbenzol in 600 ml Tetrahydrofuran/tert.-Butanol 1:1 wird bei 0 - 5°C eine Lösung von 56,6 g (0,51 Mol) Kalium-tert.-butylat in 500 ml tert.-Butanol innerhalb 1 h unter heftigem Rühren zugetropft. Nach 1 h bei 0°C wird innerhalb 2 h auf Raumtemperatur (20°C) erwärmt, darauf mit Eisessig angesäuert (pH 4), mit 2 l Wasser verdünnt und 3mal mit je 50 ml Diethylether extrahiert. Die vereinigten Etherextrakte werden mit gesättigter NaCl-Lösung gewaschen, über $MgSO_4$ getrocknet, eingeengt und der Rückstand mit Ethanol ausgerührt; man erhält 72 g (58 % der Theorie)

Verbindung 1.239, gelbe Kristalle, Schmp. 165-167°C

Beispiel 2

Zu einer Lösung von 3,4 g (0,01 Mol) 3-Chlor-5-cyano-2-(2-nitro-4-trifluormethylanilino)-pyridin (Verbindung 3,58) in 25 ml Tetrahydrofuran werden 0,24 g (0,01 Mol) Natriumhydrid portionsweise zugegeben. Nach 15 Min. werden bei Raumtemperatur 3,5 g (0,02 Mol) Benzolsulfonsäurechlorid zugetropft. Nach 48 h bei 60°C wird mit 150 ml Wasser aufgefüllt und 3mal mit je 100 ml Ether extrahiert. Die vereinigten Etherextrakte werden mit gesättigter NaCl-Lösung gewaschen, über $MgSO_4$ getrocknet und eingeengt. Der Rückstand wird chromatographiert (Kieselgel, Cyclohexan, Essigsäureethylester 9:1). Man erhält 1,5 g (31 % der Theorie) Verbindung 5.12, gelbe Kristalle, Schmp. 147-158° C.

Entsprechend dieser Herstellungsvorschriften können die in den folgenden Tabellen beschriebenen Verbindungen hergestellt werden.

Tabelle 1: 3-Cyanopyridinderivate

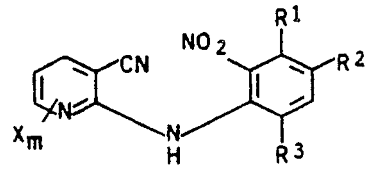

| Nr. | $X_m$ | $R^1$ | $R^2$ | $R^3$ | Schmp (°C) |
|---|---|---|---|---|---|
| 1.1 | H | Cl | $CF_3$ | $NO_2$ | 157-158 |
| 1.2 | H | Cl | CN | $NO_2$ | |
| 1.3 | H | Cl | $COOC_2H_5$ | $NO_2$ | |
| 1.4 | H | Cl | $CONH_2$ | $NO_2$ | |
| 1.5 | H | Cl | COOH | $NO_2$ | |
| 1.6 | H | F | $CF_3$ | $NO_2$ | |
| 1.7 | H | $OCH_3$ | $CF_3$ | $NO_2$ | |
| 1.8 | H | H | $CF_3$ | $NO_2$ | |
| 1.9 | H | H | CN | $NO_2$ | |
| 1.10 | H | H | Cl | $NO_2$ | |
| 1.11 | H | Cl | Cl | $NO_2$ | |
| 1.12 | H | H | $SO_2CH_3$ | $NO_2$ | |
| 1.13 | H | H | $COOCH_3$ | $NO_2$ | |
| 1.14 | H | H | $CH_3$ | $NO_2$ | |
| 1.15 | H | H | $NO_2$ | $CF_3$ | 112-115 |
| 1.16 | H | H | $NO_2$ | Cl | |
| 1.17 | H | H | $NO_2$ | $CH(CH_3)C_2H_5$ | |
| 1.18 | H | Cl | Cl | Cl | |
| 1.19 | 5-Cl | Cl | $CF_3$ | $NO_2$ | 155-156 |
| 1.20 | 5-Cl | F | $CF_3$ | $NO_2$ | |
| 1.21 | 5-Cl | Br | $CF_3$ | $NO_2$ | |
| 1.22 | 5-Cl | $CF_3$ | $CF_3$ | $NO_2$ | |
| 1.23 | 5-Cl | $OCH_3$ | $CF_3$ | $NO_2$ | |
| 1.24 | 5-Cl | $OC_2H_5$ | $CF_3$ | $NO_2$ | |
| 1.25 | 5-Cl | $OC_4H_9$(n) | $CF_3$ | $NO_2$ | |
| 1.26 | 5-Cl | $OCF_3$ | $CF_3$ | $NO_2$ | |
| 1.27 | 5-Cl | $OCF_2CHF_2$ | $CF_3$ | $NO_2$ | |
| 1.28 | 5-Cl | $OC_6H_5$ | $CF_3$ | $NO_2$ | |
| 1.29 | 5-Cl | $SC_6H_5$ | $CF_3$ | $NO_2$ | |
| 1.30 | 5-Cl | $OC_6H_4$-p-Cl | $CF_3$ | $NO_2$ | |
| 1.31 | 5-Cl | $SC_6H_4$-p-Cl | $CF_3$ | $NO_2$ | |
| 1.32 | 5-Cl | H | $CF_3$ | $NO_2$ | 167-168 |

Tabelle 1 (Fortsetzung)

| Nr. | $X_m$ | $R^1$ | $R^2$ | $R^3$ | Schmp (°C) |
|---|---|---|---|---|---|
| 1.33 | 5-Cl | H | H | Cl | |
| 1.34 | 5-Cl | H | Br | $NO_2$ | |
| 1.35 | 5-Cl | H | Cl | $NO_2$ | |
| 1.36 | 5-Cl | H | CN | $NO_2$ | |
| 1.37 | 5-Cl | H | $CONH_2$ | $NO_2$ | |
| 1.38 | 5-Cl | H | $CON(CH_3)_2$ | $NO_2$ | |
| 1.39 | 5-Cl | H | COOH | $NO_2$ | |
| 1.40 | 5-Cl | H | $COOCH_3$ | $NO_2$ | |
| 1.41 | 5-Cl | Cl | CN | $NO_2$ | |
| 1.42 | 5-Cl | Cl | $COOC_2H_5$ | $NO_2$ | 216-220 |
| 1.43 | 5-Cl | Cl | $CONH_2$ | $NO_2$ | |
| 1.44 | 5-Cl | Cl | COOH | $NO_2$ | |
| 1.45 | 5-Cl | H | $SO_2CH_3$ | $NO_2$ | |
| 1.46 | 5-Cl | H | $SO_2NH_2$ | $NO_2$ | |
| 1.47 | 5-Cl | H | $SO_2N(CH_3)_2$ | $NO_2$ | |
| 1.48 | 5-Cl | H | $CH_3$ | $NO_2$ | |
| 1.49 | 5-Cl | H | $C(CH_3)_3$ | $NO_2$ | |
| 1.50 | 5-Cl | H | $OCF_3$ | $NO_2$ | |
| 1.51 | 5-Cl | H | $OCF_2CHF_2$ | $NO_2$ | |
| 1.52 | 5-Cl | H | $NO_2$ | $CF_3$ | 153-155 |
| 1.53 | 5-Cl | H | $NO_2$ | Cl | 212-213 |
| 1.54 | 5-Cl | H | $NO_2$ | $CH_3$ | |
| 1.55 | 5-Cl | H | $NO_2$ | $CH(CH_3)C_2H_5$ | |
| 1.56 | 5-Cl | Cl | Cl | $NO_2$ | |
| 1.57 | 5-Cl | Cl | Cl | Cl | |
| 1.58 | 5-Cl | H | $CF_3$ | H | |
| 1.59 | 5-Cl | Cl | H | H | |
| 1.60 | $5-CH_3$ | Cl | $CF_3$ | $NO_2$ | |
| 1.61 | $5-CH_3$ | F | $CF_3$ | $NO_2$ | |
| 1.62 | $5-CH_3$ | H | $CF_3$ | $NO_2$ | |
| 1.63 | $5-CH_3$ | H | $NO_2$ | $CF_3$ | |
| 1.64 | $5-CH_3$ | H | $NO_2$ | Cl | |
| 1.65 | $5-CH_3$ | Cl | Cl | $NO_2$ | |
| 1.66 | $5-CH_3$ | Cl | CN | $NO_2$ | |
| 1.67 | $5-CH_3$ | Cl | $COOC_2H_5$ | $NO_2$ | |
| 1.68 | 5-Br | Cl | $CF_3$ | $NO_2$ | 136-138 |
| 1.69 | 5-Br | Cl | CN | $NO_2$ | |
| 1.70 | 5-Br | Cl | $COOC_2H_5$ | $NO_2$ | |
| 1.71 | 5-Br | Cl | $CONH_2$ | $NO_2$ | |

Tabelle 1 (Fortsetzung)

| Nr. | $X_m$ | $R^1$ | $R^2$ | $R^3$ | Schmp (°C) |
|---|---|---|---|---|---|
| 1.72 | 5-Br | F | $CF_3$ | $NO_2$ | |
| 1.73 | 5-Br | $OCH_3$ | $CF_3$ | $NO_2$ | |
| 1.74 | 5-Br | H | $CF_3$ | $NO_2$ | |
| 1.75 | 5-Br | H | CN | $NO_2$ | |
| 1.76 | 5-Br | H | Cl | $NO_2$ | |
| 1.77 | 5-Br | Cl | Cl | $NO_2$ | |
| 1.78 | 5-Br | H | $SO_2CH_3$ | $NO_2$ | |
| 1.79 | 5-Br | H | $COOCH_3$ | $NO_2$ | |
| 1.80 | 5-Br | H | $CH_3$ | $NO_2$ | |
| 1.81 | 5-Br | H | $NO_2$ | $CF_3$ | 154-156 |
| 1.82 | 5-Br | H | $NO_2$ | Cl | |
| 1.83 | 5-Br | H | $NO_2$ | $CH(CH_3)-C_2H_5$ | |
| 1.84 | 5-Br | Cl | Cl | Cl | |
| 1.85 | 5-$CF_3$ | Cl | $CF_3$ | $NO_2$ | |
| 1.86 | 5-$CF_3$ | F | $CF_3$ | $NO_2$ | |
| 1.87 | 5-$CF_3$ | H | $CF_3$ | $NO_2$ | |
| 1.88 | 5-$CF_3$ | H | $NO_2$ | $CF_3$ | |
| 1.89 | 5-$CF_3$ | H | $NO_2$ | Cl | |
| 1.90 | 5-$CF_3$ | Cl | Cl | $NO_2$ | |
| 1.91 | 5-$CF_3$ | Cl | CN | $NO_2$ | |
| 1.92 | 5-$CF_3$ | Cl | $COOC_2H_5$ | $NO_2$ | |
| 1.93 | 6-Cl | Cl | $CF_3$ | $NO_2$ | 130-134 |
| 1.94 | 6-Cl | F | $CF_3$ | $NO_2$ | |
| 1.95 | 6-Cl | Br | $CF_3$ | $NO_2$ | |
| 1.96 | 6-Cl | $CF_3$ | $CF_3$ | $NO_2$ | |
| 1.97 | 6-Cl | $OCH_3$ | $CF_3$ | $NO_2$ | |
| 1.98 | 6-Cl | $OC_2H_5$ | $CF_3$ | $NO_2$ | |
| 1.99 | 6-Cl | O-n-$C_4H_9$ | $CF_3$ | $NO_2$ | |
| 1.100 | 6-Cl | $OCF_3$ | $CF_3$ | $NO_2$ | |
| 1.101 | 6-Cl | $OCF_2CHF_2$ | $CF_3$ | $NO_2$ | |
| 1.102 | 6-Cl | $OC_6H_5$ | $CF_3$ | $NO_2$ | |
| 1.103 | 6-Cl | $SC_6H_5$ | $CF_3$ | $NO_2$ | |
| 1.104 | 6-Cl | $OC_6H_4$-p-Cl | $CF_3$ | $NO_2$ | |
| 1.105 | 6-Cl | $SC_6H_4$-p-Cl | $CF_3$ | $NO_2$ | |
| 1.106 | 6-Cl | H | $CF_3$ | $NO_2$ | |
| 1.107 | 6-Cl | H | H | Cl | |
| 1.108 | 6-Cl | H | Br | $NO_2$ | |
| 1.109 | 6-Cl | H | Cl | $NO_2$ | |
| 1.110 | 6-Cl | H | CN | $NO_2$ | |

Tabelle 1 (Fortsetzung)

| Nr. | $X_m$ | $R^1$ | $R^2$ | $R^3$ | Schmp (°C) |
|---|---|---|---|---|---|
| 1.111 | 6-Cl | H | $CONH_2$ | $NO_2$ | |
| 1.112 | 6-Cl | H | $CON(CH_3)_2$ | $NO_2$ | |
| 1.113 | 6-Cl | H | COOH | $NO_2$ | |
| 1.114 | 6-Cl | H | $COOCH_3$ | $NO_2$ | |
| 1.115 | 6-Cl | Cl | CN | $NO_2$ | |
| 1.116 | 6-Cl | Cl | $COOC_2H_5$ | $NO_2$ | |
| 1.117 | 6-Cl | Cl | $CONH_2$ | $NO_2$ | |
| 1.118 | 6-Cl | Cl | COOH | $NO_2$ | |
| 1.119 | 6-Cl | H | $SO_2CH_3$ | $NO_2$ | |
| 1.120 | 6-Cl | H | $SO_2NH_2$ | $NO_2$ | |
| 1.121 | 6-Cl | H | $SO_2N(CH_3)_2$ | $NO_2$ | |
| 1.122 | 6-Cl | H | $CH_3$ | $NO_2$ | |
| 1.123 | 6-Cl | H | $C(CH_3)_3$ | $NO_2$ | |
| 1.124 | 6-Cl | H | $OCF_3$ | $NO_2$ | |
| 1.125 | 6-Cl | H | $OCF_2CHF_2$ | $NO_2$ | |
| 1.126 | 6-Cl | H | $NO_2$ | $CF_3$ | 156-159 |
| 1.127 | 6-Cl | H | $NO_2$ | Cl | |
| 1.128 | 6-Cl | H | $NO_2$ | $CH_3$ | |
| 1.129 | 6-Cl | H | $NO_2$ | $CH(CH_3)-C_2H_5$ | |
| 1.130 | 6-Cl | Cl | Cl | $NO_2$ | |
| 1.131 | 6-Cl | Cl | Cl | Cl | |
| 1.132 | 6-Cl | H | $CF_3$ | H | |
| 1.133 | 6-Br | Cl | $CF_3$ | $NO_2$ | |
| 1.134 | 6-Br | F | $CF_3$ | $NO_2$ | |
| 1.135 | 6-Br | H | $CF_3$ | $NO_2$ | |
| 1.136 | 6-Br | H | $NO_2$ | $CF_3$ | |
| 1.137 | 6-Br | H | $NO_2$ | Cl | |
| 1.138 | 6-Br | Cl | Cl | $NO_2$ | |
| 1.139 | 6-Br | Cl | CN | $NO_2$ | |
| 1.140 | 6-Br | Cl | $COOC_2H_5$ | $NO_2$ | |
| 1.141 | 6-cyclo-$C_3H_5$ | Cl | $CF_3$ | $NO_2$ | 160 |
| 1.142 | 6-cyclo-$C_3H_5$ | F | $CF_3$ | $NO_2$ | |
| 1.143 | 6-cyclo-$C_3H_5$ | H | $CF_3$ | $NO_2$ | 168-171 |
| 1.144 | 6-cyclo-$C_3H_5$ | H | $NO_2$ | $CF_3$ | 169-173 |
| 1.145 | 6-cyclo-$C_3H_5$ | H | $NO_2$ | Cl | |
| 1.146 | 6-cyclo-$C_3H_5$ | Cl | Cl | $NO_2$ | |
| 1.147 | 6-cyclo-$C_3H_5$ | Cl | CN | $NO_2$ | |
| 1.148 | 6-cyclo-$C_3H_5$ | Cl | $COOC_2H_5$ | $NO_2$ | |
| 1.149 | 6-$CH(CH_3)_2$ | Cl | $CF_3$ | $NO_2$ | 138-141 |

Tabelle 1 (Fortsetzung)

| Nr. | $X_m$ | $R^1$ | $R^2$ | $R^3$ | Schmp (°C) |
|---|---|---|---|---|---|
| 1.150 | 6-$CH(CH_3)_2$ | F | $CF_3$ | $NO_2$ | |
| 1.151 | 6-$CH(CH_3)_2$ | H | $CF_3$ | $NO_2$ | 136 |
| 1.152 | 6-$CH(CH_3)_2$ | H | $NO_2$ | $CF_3$ | 125-131 |
| 1.153 | 6-$CH(CH_3)_2$ | H | $NO_2$ | Cl | |
| 1.154 | 6-$CH(CH_3)_2$ | Cl | Cl | $NO_2$ | |
| 1.155 | 6-$CH(CH_3)_2$ | Cl | CN | $NO_2$ | |
| 1.156 | 6-$CH(CH_3)_2$ | Cl | $COOC_2H_5$ | $NO_2$ | |
| 1.157 | 6-$CH_3$ | Cl | $CF_3$ | $NO_2$ | 163 |
| 1.158 | 6-$CH_3$ | F | $CF_3$ | $NO_2$ | |
| 1.159 | 6-$CH_3$ | H | $CF_3$ | $NO_2$ | 204-206 |
| 1.160 | 6-$CH_3$ | H | $NO_2$ | $CF_3$ | 131-132 |
| 1.161 | 6-$CH_3$ | H | $NO_2$ | Cl | |
| 1.162 | 6-$CH_3$ | Cl | Cl | $NO_2$ | |
| 1.163 | 6-$CH_3$ | Cl | CN | $NO_2$ | |
| 1.164 | 6-$CH_3$ | Cl | $COOC_2H_5$ | $NO_2$ | |
| 1.165 | 6-CN | Cl | $CF_3$ | $NO_2$ | |
| 1.166 | 6-CN | F | $CF_3$ | $NO_2$ | |
| 1.167 | 6-CN | H | $CF_3$ | $NO_2$ | |
| 1.168 | 6-CN | H | $NO_2$ | $CF_3$ | |
| 1.169 | 6-CN | H | $NO_2$ | Cl | |
| 1.170 | 6-CN | Cl | Cl | $NO_2$ | |
| 1.171 | 6-CN | Cl | CN | $NO_2$ | |
| 1.172 | 6-CN | Cl | $COOC_2H_5$ | $NO_2$ | |
| 1.173 | 6-$SC_6H_5$ | Cl | $CF_3$ | $NO_2$ | |
| 1.174 | 6-$SC_6H_5$ | F | $CF_3$ | $NO_2$ | |
| 1.175 | 6-$SC_6H_5$ | H | $CF_3$ | $NO_2$ | |
| 1.176 | 6-$SC_6H_5$ | H | $NO_2$ | $CF_3$ | |
| 1.177 | 6-$SC_6H_5$ | H | $NO_2$ | Cl | |
| 1.178 | 6-$SC_6H_5$ | Cl | Cl | $NO_2$ | |
| 1.179 | 6-$SC_6H_5$ | Cl | CN | $NO_2$ | |
| 1.180 | 6-$SC_6H_5$ | Cl | $COOC_2H_5$ | $NO_2$ | |
| 1.181 | 6-$SC_6H_5$ | Cl | $CF_3$ | $NO_2$ | |
| 1.182 | 6-$SC_6H_5$ | F | $CF_3$ | $NO_2$ | |
| 1.183 | 6-$SC_6H_5$ | H | $CF_3$ | $NO_2$ | |
| 1.184 | 6-$SC_6H_5$ | H | $NO_2$ | $CF_3$ | |
| 1.185 | 6-$SC_6H_5$ | H | $NO_2$ | Cl | |
| 1.186 | 6-$SC_6H_5$ | Cl | Cl | $NO_2$ | |
| 1.187 | 6-$SC_6H_5$ | Cl | CN | $NO_2$ | |
| 1.188 | 6-$SC_6H_5$ | Cl | $COOC_2H_5$ | $NO_2$ | |
| 1.189 | 6-$OC_2H_5$ | Cl | $CF_3$ | $NO_2$ | |

Tabelle 1 (Fortsetzung)

| Nr. | $X_m$ | $R^1$ | $R^2$ | $R^3$ | Schmp ($^oC$) |
|---|---|---|---|---|---|
| 1.190 | 6-$OC_2H_5$ | F | $CF_3$ | $NO_2$ | |
| 1.191 | 6-$OC_2H_5$ | H | $CF_3$ | $NO_2$ | |
| 1.192 | 6-$OC_2H_5$ | H | $NO_2$ | $CF_3$ | |
| 1.193 | 6-$OC_2H_5$ | H | $NO_2$ | Cl | |
| 1.194 | 6-$OC_2H_5$ | Cl | Cl | $NO_2$ | |
| 1.195 | 6-$OC_2H_5$ | Cl | CN | $NO_2$ | |
| 1.196 | 6-$OC_2H_5$ | Cl | $COOC_2H_5$ | $NO_2$ | |
| 1.197 | 4,6-$(CH_3)_2$ | Cl | $CF_3$ | $NO_2$ | 165 |
| 1.198 | 4,6-$(CH_3)_2$ | F | $CF_3$ | $NO_2$ | |
| 1.199 | 4,6-$(CH_3)_2$ | H | $CF_3$ | $NO_2$ | 215-216 |
| 1.200 | 4,6-$(CH_3)_2$ | H | $NO_2$ | $CF_3$ | 145-152 |
| 1.201 | 4,6-$(CH_3)_2$ | H | $NO_2$ | Cl | |
| 1.202 | 4,6-$(CH_3)_2$ | H | $C(CH_3)_3$ | $NO_2$ | 192-194 |
| 1.203 | 4,6-$(CH_3)_2$ | Cl | Cl | $NO_2$ | |
| 1.204 | 4,6-$(CH_3)_2$ | Cl | CN | $NO_2$ | |
| 1.205 | 4,6-$(CH_3)_2$ | H | CN | $NO_2$ | 203 Zers. |
| 1.206 | 4,6-$(CH_3)_2$ | Cl | $COOC_2H_5$ | $NO_2$ | |
| 1.207 | 4-$CH_3$, 6-Cl | Cl | $CF_3$ | $NO_2$ | 148-158 |
| 1.208 | 4-$CH_3$, 6-Cl | F | $CF_3$ | $NO_2$ | |
| 1.209 | 4-$CH_3$, 6-Cl | H | $CF_3$ | $NO_2$ | |
| 1.210 | 4-$CH_3$, 6-Cl | H | $NO_2$ | $CF_3$ | 128-133 |
| 1.211 | 4-$CH_3$, 6-Cl | H | $NO_2$ | Cl | |
| 1.212 | 4-$CH_3$, 6-Cl | Cl | Cl | $NO_2$ | |
| 1.213 | 4-$CH_3$, 6-Cl | Cl | CN | $NO_2$ | |
| 1.214 | 4-$CH_3$, 6-Cl | Cl | $COOC_2H_5$ | $NO_2$ | |
| 1.215 | 5-CN | Cl | $CF_3$ | $NO_2$ | |
| 1.216 | 5-CN | F | $CF_3$ | $NO_2$ | |
| 1.217 | 5-CN | H | $CF_3$ | $NO_2$ | |
| 1.218 | 5-CN | H | $NO_2$ | $CF_3$ | |
| 1.219 | 5-CN | H | $NO_2$ | Cl | |
| 1.220 | 5-CN | Cl | Cl | $NO_2$ | |
| 1.221 | 5-CN | Cl | CN | $NO_2$ | |
| 1.222 | 5-CN | Cl | $COOC_2H_5$ | $NO_2$ | |
| 1.223 | 5-CN, 6-$CH_3$ | Cl | $CF_3$ | $NO_2$ | > 220 |
| 1.224 | 5-CN, 6-$CH_3$ | F | $CF_3$ | $NO_2$ | |
| 1.225 | 5-CN, 6-$CH_3$ | H | $CF_3$ | $NO_2$ | |
| 1.226 | 5-CN, 6-$CH_3$ | H | $NO_2$ | $CF_3$ | 125 |
| 1.227 | 5-CN, 6-$CH_3$ | H | $NO_2$ | Cl | |
| 1.228 | 5-CN, 6-$CH_3$ | Cl | Cl | $NO_2$ | |
| 1.229 | 5-CN, 6-$CH_3$ | Cl | CN | $NO_2$ | |

Tabelle 1 (Fortsetzung)

| Nr. | $X_m$ | $R^1$ | $R^2$ | $R^3$ | Schmp (°C) |
|---|---|---|---|---|---|
| 1.230 | 5-CN, 6-$CH_3$ | Cl | $COOC_2H_5$ | $NO_2$ | |
| 1.231 | 4-$CH_3$, 5-CN, 6-$OC_2H_5$ | Cl | $CF_3$ | $NO_2$ | 120 |
| 1.232 | 4-$CH_3$, 5-CN, 6-$OC_2H_5$ | F | $CF_3$ | $NO_2$ | |
| 1.233 | 4-$CH_3$, 5-CN, 6-$OC_2H_5$ | H | $CF_3$ | $NO_2$ | |
| 1.234 | 4-$CH_3$, 5-CN, 6-$OC_2H_5$ | H | $NO_2$ | $CF_3$ | > 220 |
| 1.235 | 4-$CH_3$, 5-CN, 6-$OC_2H_5$ | H | $NO_2$ | Cl | |
| 1.236 | 4-$CH_3$, 5-CN, 6-$OC_2H_5$ | Cl | Cl | $NO_2$ | |
| 1.237 | 4-$CH_3$, 5-CN, 6-$OC_2H_5$ | Cl | CN | $NO_2$ | |
| 1.238 | 4-$CH_3$, 5-CN, 6-$OC_2H_5$ | Cl | $COOC_2H_5$ | $NO_2$ | |
| 1.239 | 4-$CH(CH_3)_2$, 5-CN, 6-$OC_2H_5$ | Cl | $CF_3$ | $NO_2$ | 165-167 |
| 1.240 | 4-$CH(CH_3)_2$, 5-CN, 6-$OC_2H_5$ | Cl | CN | $NO_2$ | |
| 1.241 | 4-$CH(CH_3)_2$, 5-CN, 6-$OC_2H_5$ | Cl | $COOC_2H_5$ | $NO_2$ | 173-174 |
| 1.242 | 4-$CH(CH_3)_2$, 5-CN, 6-$OC_2H_5$ | Cl | $CONH_2$ | $NO_2$ | |
| 1.243 | 4-$CH(CH_3)_2$, 5-CN, 6-$OC_2H_5$ | Cl | COOH | $NO_2$ | |
| 1.244 | 4-$CH(CH_3)_2$, 5-CN, 6-$OC_2H_5$ | F | $CF_3$ | $NO_2$ | |
| 1.245 | 4-$CH(CH_3)_2$, 5-CN, 6-$OC_2H_5$ | $OCH_3$ | $CF_3$ | $NO_2$ | |
| 1.246 | 4-$CH(CH_3)_2$, 5-CN, 6-$OC_2H_5$ | H | $CF_3$ | $NO_2$ | |
| 1.247 | 4-$CH(CH_3)_2$, 5-CN, 6-$OC_2H_5$ | H | CN | $NO_2$ | |
| 1.248 | 4-$CH(CH_3)_2$, 5-CN, 6-$OC_2H_5$ | H | Cl | $NO_2$ | |
| 1.249 | 4-$CH(CH_3)_2$, 5-CN, 6-$OC_2H_5$ | Cl | Cl | $NO_2$ | 167 |
| 1.250 | 4-$CH(CH_3)_2$, 5-CN, 6-$OC_2H_5$ | H | $SO_2CH_3$ | $NO_2$ | |
| 1.251 | 4-$CH(CH_3)_2$, 5-CN, 6-$OC_2H_5$ | H | $COOCH_3$ | $NO_2$ | |
| 1.252 | 4-$CH(CH_3)_2$, 5-CN, 6-$OC_2H_5$ | H | $CH_3$ | $NO_2$ | |
| 1.253 | 4-$CH(CH_3)_2$, 5-CN, 6-$OC_2H_5$ | H | $NO_2$ | $CF_3$ | 183-185 |
| 1.254 | 4-$CH(CH_3)_2$, 5-CN, 6-$OC_2H_5$ | H | $NO_2$ | Cl | 219-221 |
| 1.255 | 4-$CH(CH_3)_2$, 5-CN, 6-$OC_2H_5$ | H | $NO_2$ | $CH_3$<br>\|<br>$CH-C_2H_5$ | |
| 1.256 | 4-$CH(CH_3)_2$, 5-CN, 6-$OC_2H_5$ | Cl | Cl | Cl | |
| 1.257 | 5-$NO_2$ | Cl | $CF_3$ | $NO_2$ | |
| 1.258 | 5-$NO_2$ | F | $CF_3$ | $NO_2$ | |
| 1.259 | 5-$NO_2$ | H | $CF_3$ | $NO_2$ | |
| 1.260 | 5-$NO_2$ | H | $NO_2$ | $CF_3$ | |
| 1.261 | 5-$NO_2$ | H | $NO_2$ | Cl | |
| 1.262 | 5-$NO_2$ | Cl | Cl | $NO_2$ | |
| 1.263 | 5-$NO_2$ | Cl | CN | $NO_2$ | |
| 1.264 | 5-$NO_2$ | Cl | $COOC_2H_5$ | $NO_2$ | |
| 1.265 | 6-$SO_2CH_3$ | Cl | $CF_3$ | $NO_2$ | |
| 1.266 | 6-$SO_2CH_3$ | H | $NO_2$ | $CF_3$ | |
| 1.267 | 6-$OCH_3$ | Cl | $CF_3$ | $NO_2$ | |

Tabelle 1 (Fortsetzung)

| Nr. | $X_m$ | $R^1$ | $R^2$ | $R^3$ | Schmp (°C) |
|---|---|---|---|---|---|
| 1.268 | 6-$OCH_3$ | H | $NO_2$ | $CF_3$ | |
| 1.269 | 4-$CH_3$, 5-CN, 6-$OCH_3$ | Cl | $CF_3$ | $NO_2$ | |
| 1.270 | 4-$CH_3$, 5-CN, 6-$OCH_3$ | H | $NO_2$ | $CF_3$ | |
| 1.271 | 4-$CH(CH_3)_2$, 5-CN, 6-$OCH_3$ | Cl | $CF_3$ | $NO_2$ | |
| 1.272 | 4-$CH(CH_3)_2$, 5-CN, 6-$OCH_3$ | H | $NO_2$ | $CF_3$ | |
| 1.273 | 4-$C_2H_5$, 5-CN, 6-$OC_2H_5$ | Cl | $CF_3$ | $NO_2$ | |
| 1.274 | 4-$C_2H_5$, 5-CN, 6-$OC_2H_5$ | H | $NO_2$ | $CF_3$ | |
| 1.275 | 4-$CH_3$, 6-Br | Cl | $CF_3$ | $NO_2$ | |
| 1.276 | 4-$CH_3$, 6-Br | H | $NO_2$ | $CF_3$ | |
| 1.277 | 6-cyclo-$C_5H_9$ | Cl | $CF_3$ | $NO_2$ | |
| 1.278 | 6-cyclo-$C_5H_9$ | H | $NO_2$ | $CF_3$ | |
| 1.279 | 4-cyclo-$C_3H_5$, 6-Cl | Cl | $CF_3$ | $NO_2$ | |
| 1.280 | 4-cyclo-$C_3H_5$, 6-Cl | H | $NO_2$ | $CF_3$ | |
| 1.281 | 5-CN, 6-cyclo-$C_3H_5$ | Cl | $CF_3$ | $NO_2$ | |
| 1.282 | 5-CN, 6-cyclo-$C_3H_5$ | H | $NO_2$ | $CF_3$ | |
| 1.283 | 6-OPh-4-Cl | Cl | $CF_3$ | $NO_2$ | |
| 1.284 | 6-OPh-4-Cl | H | $NO_2$ | $CF_3$ | |
| 1.285 | 6-OPh-4-$CH_3$ | Cl | $CF_3$ | $NO_2$ | |
| 1.286 | 6-OPh-4-$CH_3$ | H | $NO_2$ | $CF_3$ | |
| 1.287 | 6-SPh-4-$CH_3$ | Cl | $CF_3$ | $NO_2$ | |
| 1.288 | 6-SPh-4-$CH_3$ | H | $NO_2$ | $CF_3$ | |

Tabelle 2: 4-Cyanopyridinderivate

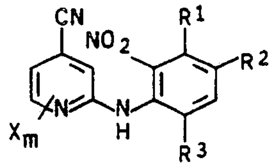

| Nr. | $X_m$ | $R^1$ | $R^2$ | $R^3$ | Schmp (°C) |
|---|---|---|---|---|---|
| 2.1 | 6-Cl | Cl | $CF_3$ | $NO_2$ | |
| 2.2 | 6-Cl | Cl | CN | $NO_2$ | |
| 2.3 | 6-Cl | Cl | $COOC_2H_5$ | $NO_2$ | |
| 2.4 | 6-Cl | Cl | $CONH_2$ | $NO_2$ | |
| 2.5 | 6-Cl | Cl | COOH | $NO_2$ | |
| 2.6 | 6-Cl | F | $CF_3$ | $NO_2$ | |
| 2.7 | 6-Cl | $OCH_3$ | $CF_3$ | $NO_2$ | |
| 2.8 | 6-Cl | H | $CF_3$ | $NO_2$ | |
| 2.9 | 6-Cl | H | CN | $NO_2$ | |
| 2.10 | 6-Cl | H | Cl | $NO_2$ | |
| 2.11 | 6-Cl | Cl | Cl | $NO_2$ | |
| 2.12 | 6-Cl | H | $SO_2CH_3$ | $NO_2$ | |
| 2.13 | 6-Cl | H | $COOCH_3$ | $NO_2$ | |
| 2.14 | 6-Cl | H | $CH_3$ | $NO_2$ | |
| 2.15 | 6-Cl | H | $NO_2$ | $CF_3$ | |
| 2.16 | 6-Cl | H | $NO_2$ | Cl | |
| 2.17 | 6-Cl | H | $NO_2$ | $CH(CH_3)-C_2H_5$ | |
| 2.18 | 6-Cl | Cl | Cl | Cl | |
| 2.19 | $6-OC_2H_5$ | Cl | $CF_3$ | $NO_2$ | |
| 2.20 | $6-OC_2H_5$ | F | $CF_3$ | $NO_2$ | |
| 2.21 | $6-OC_2H_5$ | H | $CF_3$ | $NO_2$ | |
| 2.22 | $6-OC_2H_5$ | H | $NO_2$ | $CF_3$ | |
| 2.23 | $6-OC_2H_5$ | H | $NO_2$ | Cl | |
| 2.24 | $6-OC_2H_5$ | Cl | Cl | $NO_2$ | |
| 2.25 | $6-OC_2H_5$ | Cl | CN | $NO_2$ | |
| 2.26 | $6-OC_2H_5$ | Cl | $COOC_2H_5$ | $NO_2$ | |
| 2.27 | H | Cl | $CF_3$ | $NO_2$ | |
| 2.28 | H | F | $CF_3$ | $NO_2$ | |

Tabelle 2 (Fortsetzung)

| Nr. | $X_m$ | $R^1$ | $R^2$ | $R^3$ | Schmp (°C) |
|---|---|---|---|---|---|
| 2.29 | H | H | $CF_3$ | $NO_2$ | |
| 2.30 | H | H | $NO_2$ | $CF_3$ | |
| 2.31 | H | H | $NO_2$ | Cl | |
| 2.32 | H | Cl | Cl | $NO_2$ | |
| 2.33 | H | Cl | CN | $NO_2$ | |
| 2.34 | H | Cl | $COOC_2H_5$ | $NO_2$ | |
| 2.35 | 6-$OC_6H_5$ | Cl | $CF_3$ | $NO_2$ | |
| 2.36 | 6-$OC_6H_5$ | F | $CF_3$ | $NO_2$ | |
| 2.37 | 6-$OC_6H_5$ | H | $CF_3$ | $NO_2$ | |
| 2.38 | 6-$OC_6H_5$ | H | $NO_2$ | $CF_3$ | |
| 2.39 | 6-$OC_6H_5$ | H | $NO_2$ | Cl | |
| 2.40 | 6-$OC_6H_5$ | Cl | Cl | $NO_2$ | |
| 2.41 | 6-$OC_6H_5$ | Cl | CN | $NO_2$ | |
| 2.42 | 6-$OC_6H_5$ | Cl | $COOC_2H_5$ | $NO_2$ | |
| 2.43 | 6-Br | Cl | $CF_3$ | $NO_2$ | |
| 2.44 | 6-Br | H | $NO_2$ | $CF_3$ | |
| 2.45 | 6-F | Cl | $CF_3$ | $NO_2$ | |
| 2.46 | 6-F | H | $NO_2$ | $CF_3$ | |
| 2.47 | 6-$SC_6H_5$ | Cl | $CF_3$ | $NO_2$ | |
| 2.48 | 6-$SC_6H_5$ | H | $NO_2$ | $CF_3$ | |
| 2.49 | 6-$SO_2CH_3$ | Cl | $CF_3$ | $NO_2$ | |
| 2.50 | 6-$SO_2CH_3$ | H | $NO_2$ | $CF_3$ | |
| 2.51 | 6-CN | Cl | $CF_3$ | $NO_2$ | |
| 2.52 | 6-CN | H | $NO_2$ | $CF_3$ | |

Tabelle 3: 5-Cyanopyridinderivate

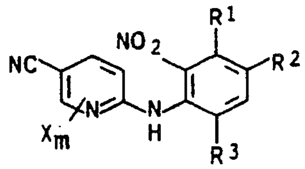

| Nr. | $X_m$ | $R^1$ | $R^2$ | $R^3$ | Schmp (°C) |
|---|---|---|---|---|---|
| 3.1 | H | Cl | $CF_3$ | $NO_2$ | 159-161 |
| 3.2 | H | Cl | CN | $NO_2$ | |
| 3.3 | H | Cl | $COOC_2H_5$ | $NO_2$ | |
| 3.4 | H | Cl | $CONH_2$ | $NO_2$ | |
| 3.5 | H | Cl | COOH | $NO_2$ | |
| 3.6 | H | F | $CF_3$ | $NO_2$ | |
| 3.7 | H | $OCH_3$ | $CF_3$ | $NO_2$ | |
| 3.8 | H | H | $CF_3$ | $NO_2$ | |
| 3.9 | H | H | CN | $NO_2$ | |
| 3.10 | H | H | Cl | $NO_2$ | |
| 3.11 | H | Cl | Cl | $NO_2$ | |
| 3.12 | H | H | $SO_2CH_3$ | $NO_2$ | |
| 3.13 | H | H | $COOCH_3$ | $NO_2$ | |
| 3.14 | H | H | $CH_3$ | $NO_2$ | |
| 3.15 | H | H | $NO_2$ | $CF_3$ | 141-144 |
| 3.16 | H | H | $NO_2$ | Cl | |
| 3.17 | H | H | $NO_2$ | $CH_3$<br>$CH-C_2H_5$ | |
| 3.18 | H | Cl | Cl | Cl | |
| 3.19 | 3-Cl | Cl | $CF_3$ | $NO_2$ | 173-175 |
| 3.20 | 3-Cl | F | $CF_3$ | $NO_2$ | 133-136 |
| 3.21 | 3-Cl | Br | $CF_3$ | $NO_2$ | |
| 3.22 | 3-Cl | $CF_3$ | $CF_3$ | $NO_2$ | |
| 3.23 | 3-Cl | $OCH_3$ | $CF_3$ | $NO_2$ | |
| 3.24 | 3-Cl | $OC_2H_5$ | $CF_3$ | $NO_2$ | |
| 3.25 | 3-Cl | $O-n-C_4H_9$ | $CF_3$ | $NO_2$ | |
| 3.26 | 3-Cl | $OCF_3$ | $CF_3$ | $NO_2$ | |
| 3.27 | 3-Cl | $OCF_2CHF_2$ | $CF_3$ | $NO_2$ | |
| 3.28 | 3-Cl | $OC_6H_5$ | $CF_3$ | $NO_2$ | |
| 3.29 | 3-Cl | $SC_6H_5$ | $CF_3$ | $NO_2$ | |
| 3.30 | 3-Cl | $OC_6H_4$-p-Cl | $CF_3$ | $NO_2$ | |
| 3.31 | 3-Cl | $SC_6H_4$-p-Cl | $CF_3$ | $NO_2$ | |
| 3.32 | 3-Cl | H | $CF_3$ | $NO_2$ | 134-135 |
| 3.33 | 3-Cl | H | H | Cl | |

Tabelle 3 (Fortsetzung)

| Nr. | $X_m$ | $R^1$ | $R^2$ | $R^3$ | Schmp (°C) |
|---|---|---|---|---|---|
| 3.34 | 3-Cl | H | Br | $NO_2$ | |
| 3.35 | 3-Cl | H | Cl | $NO_2$ | |
| 3.36 | 3-Cl | H | CN | $NO_2$ | 210-212 |
| 3.37 | 3-Cl | H | $CONH_2$ | $NO_2$ | |
| 3.38 | 3-Cl | H | $CON(CH_3)_2$ | $NO_2$ | |
| 3.39 | 3-Cl | H | COOH | $NO_2$ | |
| 3.40 | 3-Cl | H | $COOCH_3$ | $NO_2$ | 185-187 |
| 3.41 | 3-Cl | Cl | CN | $NO_2$ | |
| 3.42 | 3-Cl | Cl | $COOC_2H_5$ | $NO_2$ | 182-188 |
| 3.43 | 3-Cl | Cl | $CONH_2$ | $NO_2$ | |
| 3.44 | 3-Cl | Cl | COOH | $NO_2$ | |
| 3.45 | 3-Cl | H | $SO_2CH_3$ | $NO_2$ | mehr als 220 |
| 3.46 | 3-Cl | H | $SO_2NH_2$ | $NO_2$ | |
| 3.47 | 3-Cl | H | $SO_2N(CH_3)_2$ | $NO_2$ | |
| 3.48 | 3-Cl | H | $CH_3$ | $NO_2$ | 155 |
| 3.49 | 3-Cl | H | $C(CH_3)_3$ | $NO_2$ | 175-178 |
| 3.50 | 3-Cl | H | $OCF_3$ | $NO_2$ | |
| 3.51 | 3-Cl | H | $OCF_2CHF_2$ | $NO_2$ | |
| 3.52 | 3-Cl | H | $NO_2$ | $CF_3$ | 117-119 |
| 3.53 | 3-Cl | H | $NO_2$ | Cl | 135-138 |
| 3.54 | 3-Cl | H | $NO_2$ | $CH_3$ | |
| 3.55 | 3-Cl | H | $NO_2$ | $CH(CH_3)-C_2H_5$ | 134-138 |
| 3.56 | 3-Cl | Cl | Cl | $NO_2$ | |
| 3.57 | 3-Cl | Cl | Cl | Cl | 157-158 |
| 3.58 | 3-Cl | H | $CF_3$ | H | 164-170 |
| 3.59 | 3-Cl | Cl | H | H | |
| 3.60 | 3-$NO_2$ | Cl | $CF_3$ | $NO_2$ | |
| 3.61 | 3-$NO_2$ | F | $CF_3$ | $NO_2$ | |
| 3.62 | 3-$NO_2$ | H | $CF_3$ | $NO_2$ | |
| 3.63 | 3-$NO_2$ | H | $NO_2$ | $CF_3$ | |
| 3.64 | 3-$NO_2$ | H | $NO_2$ | Cl | |
| 3.65 | 3-$NO_2$ | Cl | Cl | $NO_2$ | |
| 3.66 | 3-$NO_2$ | Cl | CN | $NO_2$ | |
| 3.67 | 3-$NO_2$ | Cl | $COOC_2H_5$ | $NO_2$ | |
| 3.68 | 3-Br | Cl | $CF_3$ | $NO_2$ | 151-155 |
| 3.69 | 3-Br | Cl | CN | $NO_2$ | |
| 3.70 | 3-Br | Cl | $COOC_2H_5$ | $NO_2$ | |
| 3.71 | 3-Br | Cl | $CONH_2$ | $NO_2$ | |

Tabelle 3 (Fortsetzung)

| Nr. | $X_m$ | $R^1$ | $R^2$ | $R^3$ | Schmp (°C) |
|---|---|---|---|---|---|
| 3.72 | 3-Br | F | $CF_3$ | $NO_2$ | |
| 3.73 | 3-Br | $OCH_3$ | $CF_3$ | $NO_2$ | |
| 3.74 | 3-Br | H | $CF_3$ | $NO_2$ | |
| 3.75 | 3-Br | H | CN | $NO_2$ | |
| 3.76 | 3-Br | H | Cl | $NO_2$ | |
| 3.77 | 3-Br | Cl | Cl | $NO_2$ | |
| 3.78 | 3-Br | H | $SO_2CH_3$ | $NO_2$ | |
| 3.79 | 3-Br | H | $COOCH_3$ | $NO_2$ | |
| 3.80 | 3-Br | H | $CH_3$ | $NO_2$ | |
| 3.81 | 3-Br | H | $NO_2$ | $CF_3$ | 124-126 |
| 3.82 | 3-Br | H | $NO_2$ | Cl | |
| 3.83 | 3-Br | H | $NO_2$ | $CH(CH_3)-C_2H_5$ | |
| 3.84 | 3-Br | Cl | Cl | Cl | |
| 3.85 | $3-CF_3$ | Cl | $CF_3$ | $NO_2$ | |
| 3.86 | $3-CF_3$ | F | $CF_3$ | $NO_2$ | |
| 3.87 | $3-CF_3$ | H | $CF_3$ | $NO_2$ | |
| 3.88 | $3-CF_3$ | H | $NO_2$ | $CF_3$ | |
| 3.89 | $3-CF_3$ | H | $NO_2$ | Cl | |
| 3.90 | $3-CF_3$ | Cl | Cl | $NO_2$ | |
| 3.91 | $3-CF_3$ | Cl | CN | $NO_2$ | |
| 3.92 | $3-CF_3$ | Cl | $COOC_2H_5$ | $NO_2$ | |
| 3.93 | $4,6-(CH_3)_2$ | Cl | $CF_3$ | $NO_2$ | > 220 |
| 3.94 | $4,6-(CH_3)_2$ | F | $CF_3$ | $NO_2$ | |
| 3.95 | $4,6-(CH_3)_2$ | H | $CF_3$ | $NO_2$ | |
| 3.96 | $4,6-(CH_3)_2$ | H | $NO_2$ | $CF_3$ | |
| 3.97 | $4,6-(CH_3)_2$ | H | $NO_2$ | Cl | |
| 3.98 | $4,6-(CH_3)_2$ | Cl | Cl | $NO_2$ | |
| 3.99 | $4,6-(CH_3)_2$ | Cl | CN | $NO_2$ | |
| 3.100 | $4,6-(CH_3)_2$ | Cl | $COOC_2H_5$ | $NO_2$ | |
| 3.101 | 6-Cl | Cl | $CF_3$ | $NO_2$ | 203-204 |
| 3.102 | 6-Cl | F | $CF_3$ | $NO_2$ | |
| 3.103 | 6-Cl | H | $CF_3$ | $NO_2$ | |
| 3.104 | 6-Cl | H | $NO_2$ | $CF_3$ | 176-185 |
| 3.105 | 6-Cl | H | $NO_2$ | Cl | |
| 3.106 | 6-Cl | Cl | Cl | $NO_2$ | |
| 3.107 | 6-Cl | Cl | CN | $NO_2$ | |
| 3.108 | 6-Cl | Cl | $COOC_2H_5$ | $NO_2$ | |
| 3.109 | $3,6-Cl_2$ | Cl | $CF_3$ | $NO_2$ | |
| 3.110 | $3,6-Cl_2$ | F | $CF_3$ | $NO_2$ | |

Tabelle 3 (Fortsetzung)

| Nr. | $X_m$ | $R^1$ | $R^2$ | $R^3$ | Schmp (°C) |
|---|---|---|---|---|---|
| 3.111 | 3,6-$Cl_2$ | H | $CF_3$ | $NO_2$ | |
| 3.112 | 3,6-$Cl_2$ | H | $NO_2$ | $CF_3$ | |
| 3.113 | 3,6-$Cl_2$ | H | $NO_2$ | Cl | |
| 3.114 | 3,6-$Cl_2$ | Cl | Cl | $NO_2$ | |
| 3.115 | 3,6-$Cl_2$ | Cl | CN | $NO_2$ | |
| 3.116 | 3,6-$Cl_2$ | Cl | $COOC_2H_5$ | $NO_2$ | |
| 3.117 | 4-$CH_3$ | Cl | $CF_3$ | $NO_2$ | |
| 3.118 | 4-$CH_3$ | H | $NO_2$ | $CF_3$ | |
| 3.119 | 6-$CH_3$ | Cl | $CF_3$ | $NO_2$ | |
| 3.120 | 6-$CH_3$ | H | $NO_2$ | $CF_3$ | |
| 3.121 | 3-Cl, 6-$OC_2H_5$ | Cl | $CF_3$ | $NO_2$ | |
| 3.122 | 3-Cl, 6-$OC_2H_5$ | H | $NO_2$ | $CF_3$ | |
| 3.123 | 3-Cl, 6-$OC_6H_5$ | Cl | $CF_3$ | $NO_2$ | |
| 3.124 | 3-Cl, 6-$OC_6H_5$ | H | $NO_2$ | $CF_3$ | |
| 3.125 | 3-Cl, 6-$SO_2CH_3$ | Cl | $CF_3$ | $NO_2$ | |
| 3.126 | 3-Cl, 6-$SO_2CH_3$ | H | $NO_2$ | $CF_3$ | |

Tabelle 4: 6-Cyanopyridinderivate

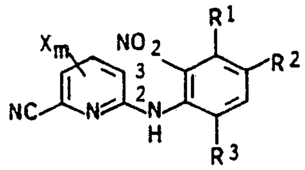

Tabelle 1 (Fortsetzung)

| Nr. | $X_m$ | $R^1$ | $R^2$ | $R^3$ | Schmp (°C) |
|---|---|---|---|---|---|
| 4.1 | H | Cl | $CF_3$ | $NO_2$ | |
| 4.2 | H | F | $CF_3$ | $NO_2$ | |
| 4.3 | H | H | $CF_3$ | $NO_2$ | |
| 4.4 | H | H | $NO_2$ | $CF_3$ | |
| 4.5 | H | H | $NO_2$ | Cl | |
| 4.6 | H | Cl | Cl | $NO_2$ | |
| 4.7 | H | Cl | CN | $NO_2$ | |
| 4.8 | H | Cl | $COOC_2H_5$ | $NO_2$ | |
| 4.9 | 3-Cl, 5-$CF_3$ | Cl | $CF_3$ | $NO_2$ | |
| 4.10 | 3-Cl, 5-$CF_3$ | F | $CF_3$ | $NO_2$ | |
| 4.11 | 3-Cl, 5-$CF_3$ | H | $CF_3$ | $NO_2$ | |
| 4.12 | 3-Cl, 5-$CF_3$ | H | $NO_2$ | $CF_3$ | |
| 4.13 | 3-Cl, 5-$CF_3$ | H | $NO_2$ | Cl | |
| 4.14 | 3-Cl, 5-$CF_3$ | Cl | Cl | $NO_2$ | |
| 4.15 | 3-Cl, 5-$CF_3$ | Cl | CN | $NO_2$ | |
| 4.16 | 3-Cl, 5-$CF_3$ | Cl | $COOC_2H_5$ | $NO_2$ | |
| 4.17 | 3,5-$Cl_2$ | Cl | $CF_3$ | $NO_2$ | |
| 4.18 | 3,5-$Cl_2$ | F | $CF_3$ | $NO_2$ | |
| 4.19 | 3,5-$Cl_2$ | H | $CF_3$ | $NO_2$ | |
| 4.20 | 3,5-$Cl_2$ | H | $NO_2$ | $CF_3$ | |
| 4.21 | 3,5-$Cl_2$ | H | $NO_2$ | Cl | |
| 4.22 | 3,5-$Cl_2$ | Cl | Cl | $NO_2$ | |
| 4.23 | 3,5-$Cl_2$ | Cl | CN | $NO_2$ | |
| 4.24 | 3,5-$Cl_2$ | Cl | $COOC_2H_5$ | $NO_2$ | |
| 4.25 | 3-$CF_3$ | Cl | $CF_3$ | $NO_2$ | |
| 4.26 | 3-$CF_3$ | F | $CF_3$ | $NO_2$ | |
| 4.27 | 3-$CF_3$ | H | $CF_3$ | $NO_2$ | |
| 4.28 | 3-$CF_3$ | H | $NO_2$ | $CF_3$ | |
| 4.29 | 3-$CF_3$ | H | $NO_2$ | Cl | |
| 4.30 | 3-$CF_3$ | Cl | Cl | $NO_2$ | |
| 4.32 | 3-$CF_3$ | Cl | $COOC_2H_5$ | $NO_2$ | |
| 4.33 | 5-$CF_3$ | Cl | $CF_3$ | $NO_2$ | |

Tabelle 4 (Fortsetzung)

| Nr. | $X_m$ | $R^1$ | $R^2$ | $R^3$ | Schmp (°C) |
|---|---|---|---|---|---|
| 4.34 | 5-$CF_3$ | F | $CF_3$ | $NO_2$ | |
| 4.35 | 5-$CF_3$ | H | $CF_3$ | $NO_2$ | |
| 4.36 | 5-$CF_3$ | H | $NO_2$ | $CF_3$ | |
| 4.37 | 5-$CF_3$ | H | $NO_2$ | Cl | |
| 4.38 | 5-$CF_3$ | Cl | Cl | $NO_2$ | |
| 4.39 | 5-$CF_3$ | Cl | CN | $NO_2$ | |
| 4.40 | 5-$CF_3$ | Cl | $COOC_2H_5$ | $NO_2$ | |
| 4.41 | 4-$CF_3$ | Cl | $CF_3$ | $NO_2$ | |
| 4.42 | 4-$CF_3$ | H | $NO_2$ | $CF_3$ | |
| 4.43 | 3-$NO_2$ | Cl | $CF_3$ | $NO_2$ | |
| 4.44 | 3-$NO_2$ | H | $NO_2$ | $CF_3$ | |
| 4.45 | 5-$NO_2$ | Cl | $CF_3$ | $NO_2$ | |
| 4.46 | 5-$NO_2$ | H | $NO_2$ | $CF_3$ | |
| 4.47 | 5-Cl, 3-$CF_3$ | Cl | $CF_3$ | $NO_2$ | |
| 4.48 | 5-Cl, 3-$CF_3$ | H | $NO_2$ | $CF_3$ | |
| 4.49 | 6-CN, 3,4,5-$Cl_3$ | Cl | $CF_3$ | $NO_2$ | |
| 4.50 | 6-CN, 3,4,5-$Cl_3$ | H | $NO_2$ | $CF_3$ | |

Tabelle 5:

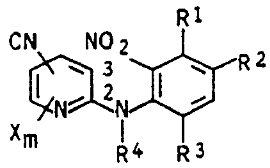

| Nr. | $X_m$/CN | $R^1$ | $R^2$ | $R^3$ | $R^4$ | Schmp. (°C) |
|---|---|---|---|---|---|---|
| 5.1 | 3-CN | Cl | $CF_3$ | $NO_2$ | $SO_2C_6H_5$ | |
| 5.2 | 3-CN | Cl | $CF_3$ | $NO_2$ | $CH_2-CH=CH_2$ | |
| 5.3 | 3-CN | Cl | $CF_3$ | $NO_2$ | $CH_2-C≡CH$ | |
| 5.4 | 3-CN | H | $NO_2$ | $CF_3$ | $COCH_2Cl$ | |
| 5.5 | 5-CN | Cl | $CF_3$ | $NO_2$ | $SO_2C_6H_5$ | |
| 5.6 | 5-CN | Cl | $CF_3$ | $NO_2$ | $CONH_2$ | |
| 5.7 | 5-CN | H | $NO_2$ | $CF_3$ | $COOCH_2C_6H_5$ | |
| 5.8 | 5-CN | H | $NO_2$ | $CF_3$ | $SO_2C_6H_5$ | |
| 5.9 | 3-Cl, 5-CN | H | H | Cl | $SO_2C_6H_5$ | |
| 5.10 | 3-Cl, 5-CN | Cl | $CF_3$ | $NO_2$ | $CH_2-CH=CH_2$ | |
| 5.11 | 3-Cl, 5-CN | Cl | $CF_3$ | $NO_2$ | $CH_2-C≡CH$ | |
| 5.12 | 3-Cl, 5-CN | H | $CF_3$ | H | $SO_2C_6H_5$ | 147-158 |
| 5.13 | 3-Cl, 5-CN | Cl | $CF_3$ | $NO_2$ | $SO_2C_6H_5$ | |
| 5.14 | 3-Cl, 5-CN | Cl | $CF_3$ | $NO_2$ | $COCH_3$ | |
| 5.15 | 3-Cl, 5-CN | Cl | $CF_3$ | $NO_2$ | $COCHCl_2$ | |
| 5.16 | 3-Cl, 5-CN | Cl | $CF_3$ | $NO_2$ | $COC_6H_5$ | |
| 5.17 | 3-Cl, 5-CN | Cl | $CF_3$ | $NO_2$ | $COOCH_3$ | |
| 5.18 | 3-Cl, 5-CN | Cl | $CF_3$ | $NO_2$ | $CONH_2$ | |
| 5.19 | 3-Cl, 5-CN | Cl | $CF_3$ | $NO_2$ | CHO | |
| 5.20 | 3-Cl, 5-CN | H | $NO_2$ | $CF_3$ | $SO_2C_6H_5$ | |
| 5.21 | 3-Cl, 5-CN | H | $NO_2$ | $CF_3$ | $COOCH_3$ | |
| 5.22 | 3-Cl, 5-CN | H | $NO_2$ | $CF_3$ | $COCH_2Cl$ | |
| 5.23 | 6-Cl, 3-CN | Cl | $CF_3$ | $NO_2$ | $SO_2C_6H_5$ | |
| 5.24 | 6-Cl, 3-CN | H | $NO_2$ | $CF_3$ | $SO_2C_6H_5$ | |
| 5.25 | 6-Cl, 5-CN | Cl | $CF_3$ | $NO_2$ | $SO_2C_6H_5$ | |
| 5.26 | 6-Cl, 5-CN | H | $NO_2$ | $CF_3$ | $SO_2C_6H_5$ | |
| 5.27 | 4-$CH(CH_3)_2$, 6-$OC_2H_5$, 3,5-$(CN)_2$ | Cl | $CF_3$ | $NO_2$ | $SO_2C_6H_5$ | |
| 5.28 | 4-$CH(CH_3)_2$, 6-$OC_2H_5$, 3,5-$(CN)_2$ | H | $NO_2$ | $CF_3$ | $SO_2C_6H_5$ | |
| 5.29 | 5-Cl, 3-CN | Cl | $CF_3$ | $NO_2$ | $SO_2C_6H_5$ | |
| 5.30 | 5-Cl, 3-CN | Cl | $CF_3$ | $NO_2$ | $COOCH_3$ | |
| 5.31 | 5-Cl, 3-CN | Cl | $CF_3$ | $NO_2$ | $CONH_2$ | |
| 5.32 | 5-Cl, 3-CN | Cl | $CF_3$ | $NO_2$ | $COC_6H_5$ | |

Tabelle 5 (Fortsetzung)

| Nr. | $X_m$/CN | $R^1$ | $R^2$ | $R^3$ | $R^4$ | Schmp. (°C) |
|---|---|---|---|---|---|---|
| 5.33 | 5-Cl, 3-CN | H | $NO_2$ | $CF_3$ | $SO_2C_6H_5$ | |
| 5.34 | 5-Cl, 3-CN | H | $NO_2$ | $CF_3$ | $COOCH_3$ | |
| 5.35 | 5-Cl, 3-CN | H | $NO_2$ | $CF_3$ | $CONH_2$ | |
| 5.36 | 5-Cl, 3-CN | H | $NO_2$ | $CF_3$ | $COC_6H_5$ | |
| 5.37 | 5-Cl, 3-CN | H | $NO_2$ | $CF_3$ | $COCHCl_2$ | |
| 5.38 | 6-cyclo-$C_3H_5$, 3-CN | Cl | $CF_3$ | $NO_2$ | $SO_2C_6H_5$ | |
| 5.39 | 6-$CH_3$, 3,5-$(CN)_2$ | Cl | $CF_3$ | $NO_2$ | $SO_2C_6H_5$ | |
| 5.40 | 6-$CH_3$, 3,5-$(CN)_2$ | H | $NO_2$ | $CF_3$ | $SO_2C_6H_5$ | |
| 5.41 | 6-Cl, 4-CN | Cl | $CF_3$ | $NO_2$ | $SO_2C_6H_5$ | |
| 5.42 | 6-Cl, 4-CN | H | $NO_2$ | $CF_3$ | $SO_2C_6H_5$ | |
| 5.43 | 4,6-$(CH_3)_2$, 3-CN | Cl | $CF_3$ | $NO_2$ | $SO_2C_6H_5$ | |
| 5.44 | 4,6-$(CH_3)_2$, 3-CN | H | $NO_2$ | $CF_3$ | $SO_2C_6H_5$ | |
| 5.45 | 4,6-$(CH_3)_2$, 3-CN | H | $CH_3$ | $NO_2$ | $SO_2C_6H_5$ | |

Tabelle 6:

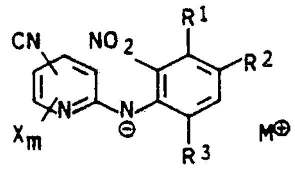

| Nr. | $X_m$/CN | $R^1$ | $R^2$ | $R^3$ | $M^+$ | Schmp. (°C) |
|---|---|---|---|---|---|---|
| 6.1 | 3-CN | Cl | $CF_3$ | $NO_2$ | $K^+$ | |
| 6.2 | 3-CN | Cl | $CF_3$ | $NO_2$ | $N(CH_3)_4^+$ | |
| 6.3 | 3-CN | Cl | $CF_3$ | $NO_2$ | $N(C_4H_9)_4^+$ | |
| 6.4 | 3-CN | Cl | $CF_3$ | $NO_2$ | $C_6H_5-NH(CH_3)_2^+$ | |
| 6.5 | 3-CN | Cl | $CF_3$ | $NO_2$ | $NH(C_2H_5)_3^+$ | |
| 6.6 | 3-CN | H | $NO_2$ | $CF_3$ | $K^+$ | |
| 6.7 | 3-CN | H | $NO_2$ | $CF_3$ | $N(CH_3)_4^+$ | |
| 6.8 | 3-CN | H | $NO_2$ | $CF_3$ | $N(C_4H_9)_4^+$ | |
| 6.9 | 5-CN | Cl | $CF_3$ | $NO_2$ | $K^+$ | |
| 6.10 | 5-CN | Cl | $CF_3$ | $NO_2$ | $N(C_4H_9)_4^+$ | |
| 6.11 | 5-CN | H | $NO_2$ | $CF_3$ | $N(C_4H_9)_4^+$ | |
| 6.12 | 3-Cl, 5-CN | Cl | $CF_3$ | $NO_2$ | $K^+$ | 175 |
| 6.13 | 3-Cl, 5-CN | Cl | $CF_3$ | $NO_2$ | $N(CH_3)_4^+$ | 198 |
| 6.14 | 3-Cl, 5-CN | Cl | $CF_3$ | $NO_2$ | $N(C_4H_9)_4^+$ | 110-112 |
| 6.15 | 3-Cl, 5-CN | Cl | $CF_3$ | $NO_2$ | $NH_2(i-C_3H_7)_2$ | 92- 94 |
| 6.16 | 3-Cl, 5-CN | Cl | $CF_3$ | $NO_2$ | $(CH_3)_3NCH_2CH_2OH^+$ | |
| 6.17 | 3-Cl, 5-CN | H | $NO_2$ | $CF_3$ | $K^+$ | |
| 6.18 | 3-Cl, 5-CN | H | $NO_2$ | $CF_3$ | $N(C_4H_9)_4^+$ | |
| 6.19 | 3-Cl, 5-CN | H | $NO_2$ | $CF_3$ | $NH_2(i-C_3H_7)_2^+$ | |
| 6.20 | 3-Br, 5-CN | Cl | $CF_3$ | $NO_2$ | $N(C_4H_9)_4^+$ | |
| 6.21 | 3-Br, 5-CN | H | $NO_2$ | $CF_3$ | $N(C_4H_9)_4^+$ | |
| 6.22 | 5-Cl, 3-CN | Cl | $CF_3$ | $NO_2$ | $K^+$ | |
| 6.23 | 5-Cl, 3-CN | Cl | $CF_3$ | $NO_2$ | $N(C_4H_9)_4^+$ | |
| 6.24 | 5-Cl, 3-CN | H | $NO_2$ | $CF_3$ | $K^+$ | |
| 6.25 | 5-Cl, 3-CN | H | $NO_2$ | $CF_3$ | $N(C_4H_9)_4^+$ | |
| 6.26 | 5-Br, 3-CN | Cl | $CF_3$ | $NO_2$ | $N(C_4H_9)_4^+$ | |
| 6.27 | 5-Br, 3-CN | H | $NO_2$ | $CF_3$ | $N(C_4H_9)_4^+$ | |
| 6.28 | 6-Cl, 3-CN | Cl | $CF_3$ | $NO_2$ | $N(C_4H_9)_4^+$ | |
| 6.29 | 6-Cl, 3-CN | H | $NO_2$ | $CF_3$ | $N(C_4H_9)_4^+$ | |

Tabelle 6 (Fortsetzung)

| Nr. | $X_m$/CN | $R^1$ | $R^2$ | $R^3$ | $M^+$ | Schmp. (°C) |
|---|---|---|---|---|---|---|
| 6.30 | 4-$CH(CH_3)_2$, 6-$OC_2H_5$, 3,5-$(CN)_2$ | Cl | $CF_3$ | $NO_2$ | $N(C_4H_9)_4^+$ | |
| 6.31 | 4-$CH(CH_3)_2$, 6-$OC_2H_5$, 3,5-$(CN)_2$ | H | $NO_2$ | $CF_3$ | $N(C_4H_9)_4^+$ | |
| 6.32 | 6-$CH_3$, 3,5-$(CN)_2$ | Cl | $CF_3$ | $NO_2$ | $N(C_4H_9)_4^+$ | |
| 6.33 | 6-$CH_3$, 3-CN | Cl | $CF_3$ | $NO_2$ | $N(C_4H_9)_4^+$ | |
| 6.34 | 6-cyclo-$C_3H_5$, 3-CN | Cl | $CF_3$ | $NO_2$ | $N(C_4H_9)_4^+$ | |
| 6.35 | 6-Cl, 4-CN | Cl | $CF_3$ | $NO_2$ | $N(C_4H_9)_4^+$ | |
| 6.36 | 6-Cl, 4-CN | H | $NO_2$ | $CF_3$ | $N(C_4H_9)_4^+$ | |
| 6.37 | 4,6-$(CH_3)_2$, 3-CN | Cl | $CF_3$ | $NO_2$ | $N(C_4H_9)_4^+$ | |
| 6.38 | 4,6-$(CH_3)_2$, 3-CN | H | $NO_2$ | $CF_3$ | $N(C_4H_9)_4^+$ | |
| 6.39 | 4,6-$(CH_3)_2$, 3-CN | H | $CH_3$ | $NO_2$ | $N(C_4H_9)_4^+$ | |
| 6.40 | 6-$CH(CH_3)_2$, 3-CN | Cl | $CF_3$ | $NO_2$ | $N(C_4H_9)_4^+$ | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch

Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1.239 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 1.207 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 3.19 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1.239 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1.226 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 1.223 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 1.234 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 1.200 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1.93 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat),
N,N′-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4, 6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithiolo-[4,5-b]-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N′,N′-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-(2-(2,4-Dichlorphenyl)-4-ethyl-1,3-dioxolan-2-yl-ethyl]-1H-1,2,4triazol
1-[2-(2,4-Dichlorphenyl)-4-n-propyl-1,3-dioxolan-2-yl-ethyl]-1H-1,2,4-triazol,

N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2′-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiele

Als Vergleichswirkstoff wurde 2-(2,4-Dinitro-6-trifluormethylanilino)-5-trifluormethylpyridin (A) - bekannt aus EP-A 31 257 - benutzt.

Anwendungsbeispiel

Wirksamkeit gegen Rebenperonospora

Blätter von Topfreben der Sorte "Müller-Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß der Wirkstoff 1.197, 1.200, 1.207, 1.223, 1.226, 1.234, 1.239 und 3.19 bei der Anwendung als 0,05 %ige (Gew.%) Spritzbrühe eine bessere fungizide Wirkung zeigen (95 - 100 %) als der bekannte Vergleichswirkstoff A (70 %).

Anwendungsbeispiel 2

Wirksamkeit gegen Botrytis cinerea

Paprikasämlinge der Sorte "Neusiedler Ideal Elite" wurden, nachdem sich 4-5 Blätter gut entwickelt

hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22-24 °C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß die Wirkstoffe 1.93 und 3.19 bei der Anwendung als 0,05 %ige (Gew.%) Spritzbrühe eine bessere fungizide Wirkung zeigen (85-95 %) als der bekannte Vergleichswirkstoff A (0 %).

## Ansprüche

1. 2-Anilino-cyanopyridine der Formel I,

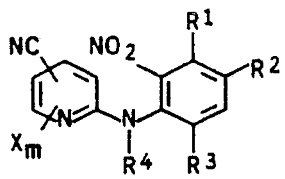

I

in welcher

X

$NO_2$, CN, Halogen, $SO_2$-$C_1$-$C_4$-Alkyl, $C_1$-$C_4$-Alkyl, $C_3$-$C_6$-Cycloalkyl, $C_1$-$C_4$-Halogenalkyl, $C_1$-$C_4$-Alkoxy, gegebenenfalls im Phenylrest substituiertes Phenoxy oder Phenylthio steht,

m

eine ganze Zahl von 1-3 ist, wobei die einzelnen Reste gleich oder verschieden sind, wenn m größer als 1 ist,

$R^1$

Wasserstoff, Halogen, $C_1$-$C_4$-Alkoxy, $C_1$-$C_4$-Halogenalkyl, $C_1$-$C_4$-Halogenalkoxy, gegebenenfalls im Phenylrest substituiertes Phenoxy oder Phenylthio ist,

$R^2$, $R^3$

unabhängig voneinander Wasserstoff, $NO_2$, Halogen, CN, $C_1$-$C_6$-Alkyl, $SO_2$-$C_1$-$C_4$-Alkyl, $C_1$-$C_6$-Halogenalkyl, $SO_2NR^5R^6$, $C_1$-$C_4$-Halogenalkoxy, $COOR^5$, $CONR^5R^6$ ist,

$R^4$

Wasserstoff, $COOR^7$, $CONR^5R^6$, CHO, $COR^7$, $SO_2R^7$, $C_3$-$C_6$-Alkenyl, $C_3$-$C_6$-Alkinyl ist,

$R^5$, $R^6$

unabhängig voneinander Wasserstoff, $C_1$-$C_4$-Alkyl ist,

$R^7$

$C_1$-$C_4$-Alkyl, $C_1$-$C_4$-Halogenalkyl, Benzyl, gegebenenfalls substituiertes Aryl ist,

und Salze dieser 2-Anilino-cyanopyridine der Formel I, in der $R^4$ Wasserstoff bedeutet, mit pflanzenverträglichen Kationen.

2. Verfahren zur Herstellung der Cyanopyridine der Formel I gemäß Anspruch 1, in der $R^4$ Wasserstoff ist, dadurch gekennzeichnet, daß man ein Cyanopyridin der Formel II, in der X und m die in Anspruch 1 angegebenen Bedeutungen haben, mit Aromaten der Formel III,

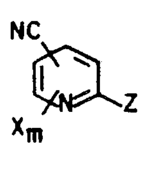

II

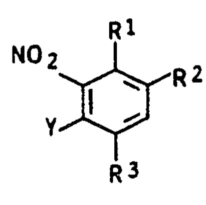

III

in der $R^1$, $R^2$, $R^3$ die in Anspruch 1 angegebenen Bedeutungen haben, gegebenenfalls in Anwesenheit einer organischen oder anorganischen Base umsetzt, wobei Y und Z für $NH_2$ oder Halogen stehen und Y und Z jeweils voneinander verschieden sind.

3. Verfahren zur Herstellung der Cyanopyridine der Formel I gemäß Anspruch 1, in der $R^4$ verschieden

von Wasserstoff ist, dadurch gekennzeichnet, daß man ein Cyanopyridin der Formel I, in der $R^4$ Wasserstoff ist und X, m, $R^1$, $R^2$, $R^{3'}$ $R^{5'}$ $R^6$, $R^7$, die in Anspruch 1 angegebenen Bedeutungen haben, mit einem Alkylierungs-, Acylierungs- oder Sulfonylierungsmittel der Formel IV

$LR^4$ IV,

in der $R^4$ die in Anspruch 1 angegebene Bedeutung außer Wasserstoff hat und L für eine nucleophil verdrängbare Abgangsgruppe steht, gegebenenfalls in Gegenwart einer organischen oder anorganischen Base umsetzt.

4. Fungizides Mittel, enthaltend eine fungizid wirksame Menge einer Verbindung der Formel I,

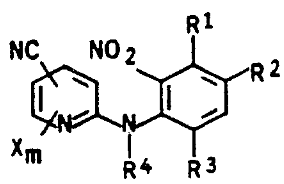

I

in welcher

X

$NO_2$, CN, Halogen, $SO_2$-$C_1$-$C_4$-Alkyl, $C_1$-$C_4$-Alkyl, $C_3$-$C_6$-Cycloalkyl, $C_1$-$C_4$-Halogenalkyl, $C_1$-$C_4$-Alkoxy, gegebenenfalls im Phenylrest substituiertes Phenoxy oder Phenylthio steht,

m

eine ganze Zahl von 1-3 ist, wobei die einzelnen Reste gleich oder verschieden sind, wenn m größer als 1 ist,

$R^1$

Wasserstoff, Halogen, $C_1$-$C_4$-Alkoxy, $C_1$-$C_4$-Halogenalkyl, $C_1$-$C_4$-Halogenalkoxy, gegebenenfalls im Phenylrest substituiertes Phenoxy oder Phenylthio ist,

$R^2$, $R^3$

unabhängig voneinander Wasserstoff, $NO_2$, Halogen, CN, $C_1$-$C_6$-Alkyl, $SO_2$-$C_1$-$C_4$-Alkyl, $C_1$-$C_6$-Halogenalkyl, $SO_2NR^5R^6$, $C_1$-$C_4$-Halogenalkoxy, $COOR^5$, $CONR^5R^6$ ist,

$R^4$

Wasserstoff, $COOR^7$, $CONR^5R^6$, CHO, $COR^7$, $SO_2R^7$, $C_3$-$C_6$-Alkenyl, $C_3$-$C_6$-Alkinyl ist,

$R^5$, $R^6$

unabhängig voneinander Wasserstoff, $C_1$-$C_4$-Alkyl ist,

$R^7$

$C_1$-$C_4$-Alkyl, $C_1$-$C_4$-Halogenalkyl, Benzyl, gegebenenfalls substituiertes Aryl ist,
oder Salze der 2-Anilino-cyanopyridine der Formel I, in der $R^4$ Wasserstoff bedeutet, mit pflanzenverträglichen Kationen und einen festen oder flüssigen Trägerstoff.

5. Verfahren zur Herstellung von Fungiziden, dadurch gekennzeichnet, daß man eine Verbindung der Formel I gemäß Anspruch 1 mit einem festen oder flüssigen Trägerstoff mischt.

6. Verfahren zur Bekämpfung von Pilzen, dadurch gekennzeichnet, daß man eine fungizid wirksame Menge einer Verbindung der Formel I

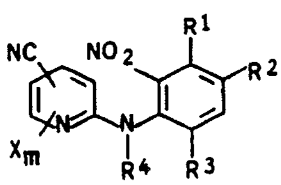

I

in welcher

X

$NO_2$, CN, Halogen, $SO_2$-$C_1$-$C_4$-Alkyl, $C_1$-$C_4$-Alkyl, $C_3$-$C_6$-Cycloalkyl, $C_1$-$C_4$-Halogenalkyl, $C_1$-$C_4$-Alkoxy, gegebenenfalls im Phenylrest substituiertes Phenoxy oder Phenylthio steht,

m

eine ganze Zahl von 1-3 ist, wobei die einzelnen Reste gleich oder verschieden sind, wenn m größer als 1 ist,

$R^1$

Wasserstoff, Halogen, $C_1$-$C_4$-Alkoxy, $C_1$-$C_4$-Halogenalkyl, $C_1$-$C_4$-Halogenalkoxy, gegebenenfalls im Phenyl-

rest substituiertes Phenoxy oder Phenylthio ist,
$R^2$, $R^3$
unabhängig voneinander Wasserstoff, $NO_2$, Halogen, CN, $C_1$-$C_6$-Alkyl, $SO_2$-$C_1$-$C_4$-Alkyl, $C_1$-$C_6$-Halogenalkyl, $SO_2NR^5R^6$, $C_1$-$C_4$-Halogenalkoxy, $COOR^5$, $CONR^5R^6$ ist,
$R^4$
Wasserstoff, $COOR^7$, $CONR^5R^6$, CHO, $COR^7$, $SO_2R^7$, $C_3$-$C_6$-Alkenyl, $C_3$-$C_6$-Alkinyl ist,
$R^5$, $R^6$
unabhängig voneinander Wasserstoff, $C_1$-$C_4$-Alkyl ist,
$R^7$
$C_1$-$C_4$-Alkyl, $C_1$-$C_4$-Halogenalkyl, Benzyl, gegebenenfalls substituiertes Aryl ist,
oder Salze der 2-Anilino-cyanopyridine der Formel I, in der $R^4$ Wasserstoff bedeutet, mit pflanzenverträglichen Kationen auf Pilze oder auf durch Pilzbefall bedrohte Pflanzen, Saatgüter, Materialien oder den Erdboden einwirken läßt.

7. Verbindung der Formel I gemäß Anspruch 1, dadurch gekennzeichnet, daß $X_m$ 4-iso-Propyl, 3-Cyan, 5-Cyan, 6-Ethoxy, $R^1$ Chlor, $R^2$ Trifluormethyl, $R^3$ Nitro und $R^4$ Wasserstoff bedeutet.

8. Verbindung der Formel I gemäß Anspruch 1, dadurch gekennzeichnet, daß $X_m$ 3-Chlor, 5-Cyan, $R^1$ Wasserstoff, $R^2$ Trifluormethyl, $R^3$ Wasserstoff und $R^4$ Benzolsulfonyl bedeutet.

Europäisches Patentamt

# EUROPÄISCHER RECHERCHENBERICHT

Nummer der Anmeldung

EP 90 10 3017

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 291 809 (BASF AG)<br>* Seite 3, Zeilen 1-42 *<br>--- | 1,5 | C 07 D 213/84<br>A 01 N 43/42 |
| A | EP-A-0 013 145 (ICI)<br>* Seite 50, Anspruch 1, Seite 57, Anspruch 10 *<br>--- | 1,5 | |
| A,D | EP-A-0 031 257 (ISHIHARA SANGYO KAISHA)<br>* Seiten 2,3; Zusammenfassung *<br>--- | 1,5 | |
| A | PATENT ABSTRACTS OF JAPAN<br>Band 9, Nr. 196 (C-297)(1919), 13. August 1985; & JP - A - 60 64963 (ISHIHARA SANGYO) 13.04.1985<br>----- | 1,5 | |
| | | | **RECHERCHIERTE SACHGEBIETE (Int. Cl.5)** |
| | | | C 07 D 213/00 |

Der vorliegende Recherchenbericht wurde für alle Patentansprüche erstellt

| Recherchenort | Abschlußdatum der Recherche | Prüfer |
|---|---|---|
| BERLIN | 09-05-1990 | KYRIAKAKOU G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)